# FASCICULE DE BREVET EUROPEEN

(11) **EP 3 164 178 B1**
(45) Date de publication et mention de la délivrance du brevet: **04.09.2019**
(21) Numéro de dépôt: 15748277.9
(22) Date de dépôt: 02.07.2015
(51) Int. Cl.: A61M 5/20, A61M 5/315, A61M 5/32, A61M 5/46

(54) **AUTOINJECTEUR**
AUTOINJEKTOR
AUTOINJECTOR

(30) Priorité: 04.07.2014 FR 1456445
(43) Date de publication de la demande: 10.05.2017
(73) Titulaire: APTAR France SAS, 27110 Le Neubourg (FR)
(72) Inventeur: FABIEN, David, F-22700 Saint Quay Perros (FR)
(74) Mandataire: CAPRI
(86) Numéro de dépôt international: PCT/FR2015/051833
(87) Numéro de publication internationale: WO 2016/001595

(56) Documents cités:
- EP-A1- 2 705 862
- WO-A1-2011/101382
- WO-A1-2015/001280
- WO-A2-2009/010591
- FR-A1- 2 654 938

## Description

La présente invention concerne un autoinjecteur.

Les autoinjecteurs sont bien connus dans l'état de la technique. Ces dispositifs ont principalement pour but de réaliser une injection automatique du contenu d'une seringue à l'intérieur du corps d'un patient. Divers systèmes existent pour rendre automatique la pénétration de l'aiguille dans le corps du patient ainsi que l'injection du produit fluide contenu dans la seringue. Les autoinjecteurs sont des dispositifs relativement complexes qui doivent répondre à un certain nombre d'exigences de contraintes pour être fiables. La robustesse du dispositif, sa maniabilité, et sa facilité d'utilisation pour l'utilisateur sont également des éléments importants. Par ailleurs, la plupart de ces autoinjecteurs étant à usage unique, le coût de fabrication et d'assemblage est également un facteur dont il faut tenir compte.

Il existe de nombreux autoinjecteurs sur le marché, qui présentent toutefois tous un certain nombre d'inconvénients.

Des autoinjecteurs de ce genre sont divulgués par les documents EP 2 705 862 A1, FR 2 654 938 A1, WO 2009/010591 A2 et WO 2011/101382 A1.

Ainsi, pour les autoinjecteurs qui utilisent le même ressort pour d'abord effectuer le piquage puis l'injection, le ressort doit être suffisamment puissant pour garantir la totalité de la phase d'injection. Ceci est d'autant plus vrai qu'au début de la phase d'injection, il faut généralement une force relativement importante pour décoller le piston de la seringue. De ce fait, le ressort exprime sa puissance maximale lors du piquage, ce qui peut rendre cette phase de piquage douloureuse. De plus, avec un tel ressort très puissant lors du piquage, il existe un risque important de casse de la collerette de la seringue, en particulier quand il s'agit de seringue en verre.

Par ailleurs, il est souhaitable d'éviter tout risque de déclenchement non souhaité de l'autoinjecteur, par exemple pendant son stockage ou son transport, sans rendre toutefois l'actionnement du dispositif trop complexe ou difficile.

La présente invention a pour but de fournir un autoinjecteur qui ne reproduit pas les inconvénients susmentionnés, et qui permet de répondre aux différentes exigences et contraintes importantes pour une utilisation sûre et fiable de l'autoinjecteur.

La présente invention a aussi pour but de fournir un autoinjecteur qui soit fiable et sûr d'utilisation, qui permette de garantir la distribution de la totalité du produit fluide à l'endroit souhaité, et qui soit simple et peu coûteux à fabriquer et à assembler.

La présente invention a donc pour objet un autoinjecteur comportant un corps, un réservoir contenant du produit fluide et comportant un piston et une aiguille, tel qu'une seringue pré-remplie, ledit autoinjecteur comportant une tige de piston adaptée à coopérer avec le piston dudit réservoir, ladite tige de piston étant déplaçable entre une position de repos et une position d'injection dans laquelle ladite tige de piston a déplacé le piston du réservoir pour injecter le produit fluide à travers l'aiguille, un ressort d'actionnement étant prévu pour solliciter ladite tige de piston vers sa position d'injection, l'autoinjecteur comprenant un système de réglage de force adapté à exercer au moins une force F2 sur ladite tige de piston, ladite force F2 s'ajoutant à la force exercée par ledit ressort d'actionnement sur ladite tige de piston en début d'injection pour amplifier la force exercée sur ledit piston par ladite tige de piston en début d'injection, ledit système de réglage de force comprenant deux organes pivotants coopérant avec ladite tige de piston, lesdits organes pivotants étant reliés entre eux par deux éléments élastiques, au moins un organe pivotant étant bloqué avant actionnement par un système de verrouillage, bloquant ainsi l'actionnement de l'autoinjecteur, ledit autoinjecteur comportant un élément de déverrouillage adapté à déplacer et/ou déformer ledit système de verrouillage pour débloquer ledit au moins un organe pivotant, et permettre ainsi l'actionnement de l'autoinjecteur.

Avantageusement, ledit corps comporte un manchon, lesdits organes pivotants étant monté pivotants sur ledit manchon autour d'axes de rotation parallèles.

Avantageusement, lesdits éléments élastiques sont fixés auxdits organes pivotants au niveau d'axes mobiles parallèles, tels que des tiges ayant deux bords latéraux, formés sur lesdits organes pivotants.

Avantageusement, lorsque la tige de piston se déplace vers sa position d'injection, lesdits axes mobiles sont disposés en arrière desdits axes fixes dans le sens de déplacement de ladite tige de piston, lesdits éléments élastiques chargés, en début de déplacement de la tige de piston vers sa position d'injection, faisant tourner lesdits organes pivotants de telle manière à détendre lesdits éléments élastiques, créant ainsi une force d'amplification F2 en début d'injection.

Avantageusement, chaque organe pivotant comprend plusieurs projections adaptées à coopérer avec plusieurs projections radiales de la tige de piston.

Avantageusement, ledit autoinjecteur est actionné par un bouton axial.

Avantageusement, avant injection, ladite tige de piston est d'abord déplacée par ledit ressort d'actionnement entre ladite position de repos et une position de piquage, dans laquelle ladite tige de piston a déplacé ledit réservoir par rapport audit corps pour réaliser le piquage.

Avantageusement, lors du piquage, la tige de piston coopère avec le piston du réservoir pour déplacer ledit réservoir par rapport au corps.

Avantageusement, lorsque la tige de piston se déplace de sa position de repos vers sa position de piquage, lesdits axes mobiles sont disposés en avant desdits axes fixes dans le sens de déplacement de ladite tige de piston, ladite tige de piston, en fin de déplacement vers sa position de piquage, faisant tourner lesdits organes pivotants de telle manière à charger lesdits éléments élastiques, créant ainsi une force de freinage F1 en fin de piquage, ladite force F1 étant opposée à la force exercée par ledit ressort d'actionnement sur ladite tige de piston en fin de piquage pour diminuer la force exercée sur ledit réservoir par ladite tige de piston en fin de piquage.

Avantageusement, ledit système de verrouillage comporte au moins un élément de verrouillage déplaçable et/ou déformable entre une position de verrouillage, dans laquelle il bloque au moins un organe pivotant en rotation, et une position de déverrouillage, dans laquelle ledit au moins un organe pivotant peut pivoter.

Avantageusement, ledit au moins un élément de verrouillage est sollicité élastiquement vers sa position de verrouillage par un organe de sollicitation, tel qu'une lame élastique.

Avantageusement, ledit au moins un élément de verrouillage est monté pivotant sur un manchon dudit corps, autour d'un axe de rotation.

Avantageusement, ledit au moins un élément de verrouillage comporte une partie de blocage qui coopère directement avec un organe pivotant et une partie de commande qui coopère avec ledit élément de déverrouillage.

Avantageusement, ledit élément de déverrouillage est formé sur un manchon actionneur, monté coulissant dans ledit corps, et dont une surface d'extrémité axiale est en contact avec la zone où l'injection doit avoir lieu, ledit manchon actionneur étant adapté à coulisser par rapport audit corps inférieur entre une position initiale de repos, avant actionnement, dans laquelle il se projette axialement hors dudit corps inférieur, une position actionnée, dans laquelle il est déplacé axialement vers l'intérieur dudit corps inférieur, et une position finale de sécurité, dans laquelle il est à nouveau projeté hors dudit corps inférieur, pour recouvrir l'aiguille de la seringue après injection, ledit manchon actionneur étant sollicité axialement vers l'extérieur dudit corps inférieur par un ressort.

Avantageusement, ledit élément de déverrouillage coopère avec ledit système de verrouillage en fin de course dudit manchon actionneur entre sa position initiale de repos et sa position actionnée, notamment lorsque ledit manchon actionneur a effectué au moins 90% de ladite course.

Ces caractéristiques et avantages et d'autres de la présente invention apparaîtront plus clairement au cours de la description détaillée suivante, faite en référence aux dessins joints, donnés à titre d'exemples non limitatifs, et sur lesquels :
La figure 1 est une vue schématique en perspective éclatée des composants d'un autoinjecteur, selon un mode de réalisation avantageux,
La figure 2 est une vue de détail en section transversale du dispositif de la figure 1,
La figure 3 est une vue de détail de côté d'une partie du dispositif de la figure 1,
La figure 4 est une vue similaire à celle de la figure 3, vue de dessous,
Les figures 5 à 9 illustrent de manière schématique, vues en section transversale, les séquences successives de la présente invention, selon le mode de réalisation de la figure 1,
Les figures 10 à 14 illustrent de manière schématique, vues de côté, les séquences successives des figures 5 à 9,
Les figures 15 à 18 illustrent schématiquement le déverrouillage des organes pivotants, en début d'actionnement, et
Les figures 19 à 22 illustrent en section transversale un autre mode de réalisation de l'invention.

L'autoinjecteur va être décrit ci-après en référence à deux modes de réalisation avantageux de celui-ci. Il est toutefois à noter que les autoinjecteurs, qui sont des appareils complexes, comprennent plusieurs modules pour réaliser plusieurs fonctions. Ces divers modules peuvent être utilisés séparément et indépendamment les uns des autres, sans être nécessairement combinés aux autres modules, et pourraient notamment être utilisés dans des autoinjecteurs de forme différente de celle représentée sur les dessins. De plus, il est à noter que les dessins sont des vues schématiques qui ne représentent pas forcément la forme exacte des composants d'un autoinjecteur, et ne sont pas forcément à l'échelle, notamment dans des buts de clarté. Par ailleurs, les dessins ne représentent pas forcément tous les éléments constitutifs d'un autoinjecteur, mais seulement les éléments nécessaires au fonctionnement de la présente invention. Ainsi, divers éléments et modules supplémentaires et/ou complémentaires pourraient être associés à l'autoinjecteur représenté sur les figures.

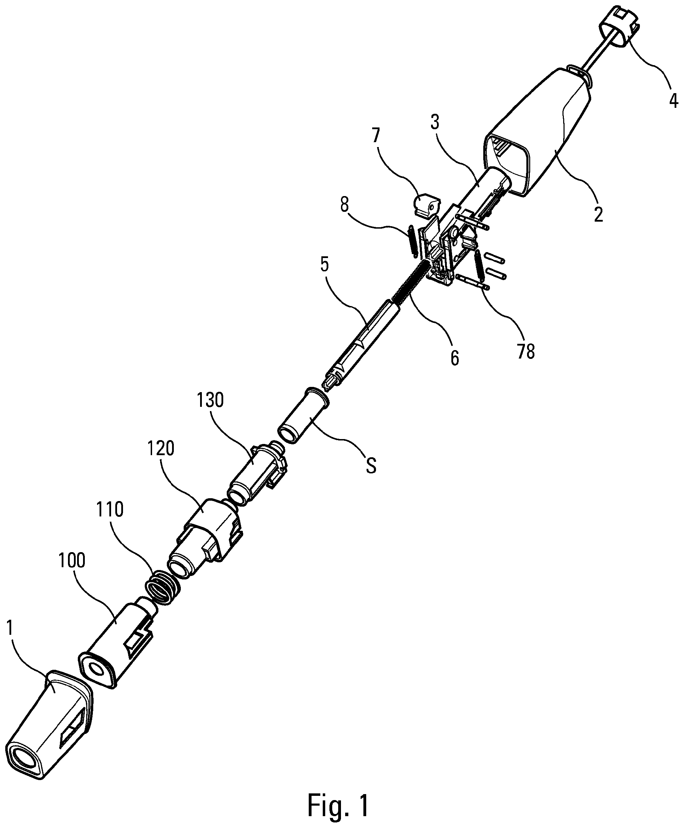

En se référant à la figure 1, les différents composants de l'autoinjecteur, selon un premier mode de réalisation avantageux, sont représentés de manière éclatée.

Dans l'ordre des références numériques, l'autoinjecteur comporte un corps inférieur 1, un corps supérieur 2 contenant un manchon 3, un bouton axial d'actionnement 4, une tige de piston 5, un ressort d'actionnement 6, et deux organes pivotants 7, interconnectés au moyen de deux éléments élastiques 8, de préférence sous forme de ressorts.

Il est à noter que les corps inférieur et supérieur pourraient être remplacés par un corps unique. De même, un corps constitué de plus de deux parties de corps est aussi envisageable.

Un réservoir S est inséré dans ledit autoinjecteur, notamment dans son corps inférieur 1. Ce réservoir S contient du produit fluide, et comporte un piston et une aiguille (non représentés dans ce mode de réalisation). Le piston est adapté à se déplacer dans ledit réservoir S pour injecter le produit fluide à travers ladite aiguille.

La présente description sera faite en référence à une seringue S, qui peut être de tout type. Plus généralement, il est entendu que le terme « seringue » dans la présente description englobe tout type de réservoir associé à une aiguille. De préférence, le réservoir S est une seringue pré-remplie.

Le corps inférieur 1 comporte à son extrémité avant (dans le sens de déplacement de la seringue S) une ouverture permettant le passage de l'aiguille lors de la phase de piquage.

Le corps inférieur 1 contient un manchon actionneur 100 dont la surface d'extrémité axiale 105 est en contact avec la partie du corps de l'utilisateur où l'injection doit se faire. Après actionnement, le manchon actionneur 100 recouvre l'aiguille de la seringue S pour éviter tout risque de piqûre avec ladite aiguille. Le manchon actionneur 100 est adapté à coulisser par rapport audit corps inférieur 1 entre une position initiale de repos, avant actionnement, dans laquelle il se projette axialement hors dudit corps inférieur 1, une position actionnée, dans laquelle il est déplacé axialement vers l'intérieur dudit corps inférieur 1, et une position finale de sécurité, dans laquelle il est à nouveau projeté hors dudit corps inférieur 1, pour recouvrir l'aiguille de la seringue S après injection. Il est à noter que la position finale de sécurité peut être identique à la position initiale de repos, ou en variante, ces deux positions peuvent être différentes, avec par exemple le manchon actionneur 100 s'étendant axialement plus loin hors dudit corps inférieur 1 dans ladite position finale de sécurité par rapport à ladite position initiale de repos. Ce manchon actionneur 100 est avantageusement sollicité axialement vers l'extérieur dudit corps inférieur 1 par un ressort 110.

Le corps inférieur 1 peut en outre contenir un corps interne 120 pouvant recevoir un élément de support de réservoir 130 dans lequel est insérée ladite seringue S.

Le corps supérieur 2 se fixe au corps inférieur 1, et il peut recevoir un manchon central 3 adapté à loger la tige de piston 5 et le ressort 6.

Le bouton axial d'actionnement 4 peut être monté coulissant axialement par rapport au corps supérieur 2, et en contact avec la tige de piston 5. Ainsi, en appuyant sur le bouton axial 4 pour l'enfoncer axialement dans le corps supérieur 2, on déplace axialement la tige de piston 5, ce qui permet l'actionnement du dispositif comme cela sera décrit ci-après. Le bouton axial pourrait en variante être remplacé par un bouton latéral.

La tige de piston 5 comporte une partie arrière 54 et une extrémité avant 55, dans le sens de déplacement de la tige de piston 5 dans le corps supérieur 2.

Dans cet exemple, la partie arrière 54 définit une partie tubulaire recevant le ressort 6 et une partie du bouton axial d'actionnement 4.

L'extrémité avant 55 a pour but de contacter le piston de la seringue S pour déplacer ledit piston et ainsi injecter le produit contenu dans la seringue S à travers l'aiguille.

La tige de piston 5 peut aussi comporter plusieurs projections radiales 52, 53. Une première projection radiale 52, proximal de l'extrémité avant 55, qui définit une surface avant 51 formant épaulement. Et une seconde projection radiale 53, décalée axialement de ladite première projection radiale 52 en direction de l'extrémité arrière 54, et qui définit une surface avant et une surface arrière. Bien sur, il ne s'agit là que d'exemples de réalisation, et un homme du métier est capable de réaliser ces épaulements et projections radiales d'une manière différente de celle représentée sur les dessins. En particulier, lesdites projections radiales 52, 53 ne sont pas nécessairement dans la partie avant de la tige de piston 5, comme représenté sur les dessins, mais elles pourraient être réalisées sur une autre partie de la tige de piston 5. Des creux pourraient aussi remplacer les projections.

Le ressort d'actionnement 6 peut s'appuier d'une part dans le manchon 3 et d'autre part sur la tige de piston, par exemple sur un quatrième épaulement 58 décalé axialement dudit troisième épaulement 53 en direction de la partie arrière 54. Dans l'exemple représenté, ce quatrième épaulement 58 forme une base de la partie tubulaire susmentionnée.

Les organes pivotants 7 sont avantageusement assemblés de manière pivotante sur le manchon 3, et ils sont avantageusement identiques. Ils sont de préférence disposés de part et d'autre de la tige de piston 5. Ils ne sont pas déplaçables axialement par rapport audit corps inférieur 1, mais seulement en pivotement autour de leurs axes de rotation 79 qui sont parallèles. En variante, ils pourraient être assemblés différemment, notamment sur le corps inférieur 1 ou sur le corps supérieur 2.

Chaque organe pivotant 7 peut comporter plusieurs projections 71, 72, 73. Une première projection 71 adaptée à coopérer avec la surface avant (dans le sens de déplacement axial de la tige de piston au cours de l'actionnement) de la première projection radiale 52 de la tige de piston 5. Une seconde projection 72 est adaptée à coopérer avec la surface avant de la seconde projection radiale 53. Et une troisième projection 73 est adaptée à coopérer avec la surface arrière de la seconde projection radiale 53. Bien entendu, d'autres mises en oeuvre sont envisageables, par exemple avec un nombre différent de projections. Des creux pourraient aussi remplacer les projections.

Les éléments élastiques 8 relient les deux organes pivotants 7 entre eux. Le principe consiste à combiner deux axes de rotation fixes, en particulier les axes de rotation 79 des deux organes pivotants 7, avec deux axes mobiles, en particulier les points d'accroche 78 des organes pivotants 7 sur lesquels se fixent les éléments élastiques 8.

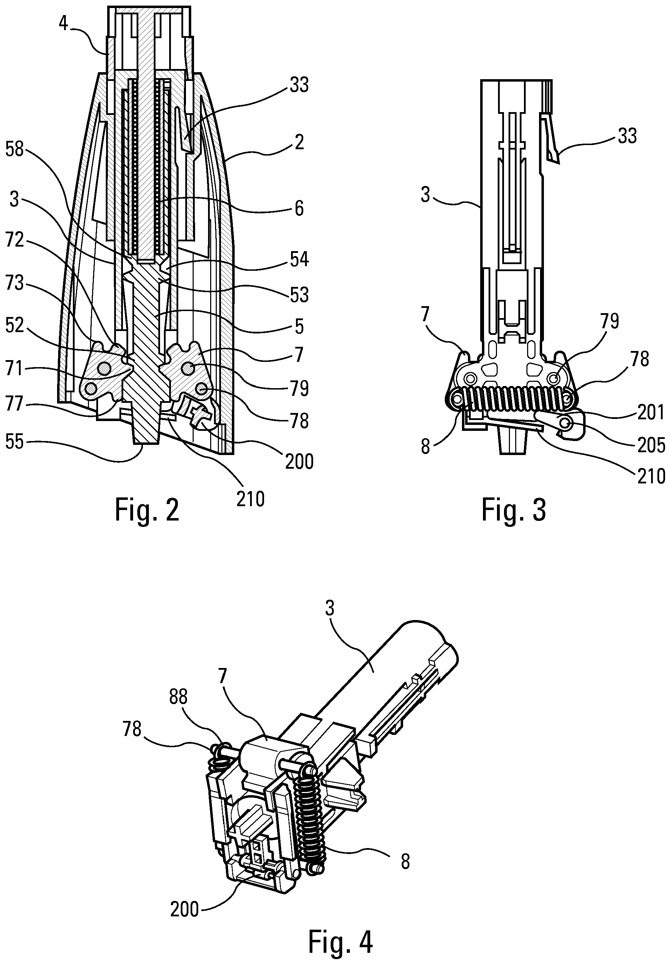

Ainsi, comme visible sur la figure 4, le premier élément élastique 8 peut se fixer d'une part sur un premier point d'accroche du premier organe pivotant et d'autre part sur un premier point d'accroche du second organe pivotant, et il peut en être de même et de manière symétrique pour l'autre élément élastique. De préférence, chaque organe pivotant 7 comporte une tige 78 ayant deux bords latéraux saillants. Le premier élément élastique relie alors les premiers bords saillants entre eux et le second élément élastique relie les seconds bords saillants entre eux. D'autres variantes de réalisation sont possibles. Avantageusement, lorsque les éléments élastiques 8 sont des ressorts, ils sont identiques et comportent des oeillets 88 adaptés à se fixer sur lesdits premiers et seconds bords saillants 78 des organes pivotants 7. En variante, les éléments élastiques pourraient être différents, par exemple sous la forme de joints toriques ou d'autres éléments en matériau élastiquement déformable. L'utilisation d'anneaux en matériau élastique, tels que de joints toriques, en remplacement des ressorts illustrés sur les dessins permettrait notamment de réduire l'encombrement radial du système.

Ainsi, lorsque les axes mobiles 78 sont décalés axialement par rapport aux axes de rotation fixes 79, ils exercent une force par l'intermédiaire des éléments élastiques 8.

Lorsque lesdits axes mobiles 78 sont disposés en avant desdits axes fixes 79 dans le sens de déplacement de la tige de piston 5, cette force va à l'encontre de la rotation impartie auxdits organes pivotants 7 par ladite tige de piston 5. Dans ce cas, la rotation des organes pivotants 7 est donc freinée par lesdits éléments élastiques 8.

Au contraire, lorsque lesdits axes mobiles 78 sont disposés en arrière desdits axes fixes 79 dans le sens de déplacement de la tige de piston 5, cette force va dans le sens de la rotation impartie auxdits organes pivotants 7 par ladite tige de piston 5. Dans ce cas, la rotation des organes pivotants 7 est donc amplifiée par lesdits éléments élastiques 8.

Lorsque les axes fixes 79 et mobiles 78 sont alignés, on se trouve à un point neutre, dans lequel lesdits éléments élastiques 8 n'influencent pas la rotation des organes pivotants 7. C'est dans cette position que le système bascule d'un état de « freinage » ou « d'amortissement » à un état « d'amplification ».

Un tel système de réglage de force est adapté à exercer une force F1 et/ou une force F2 sur ladite tige de piston 5. La force F1 est opposée à la force exercée par le ressort d'actionnement 6 sur la tige de piston 5 en fin de piquage pour diminuer la force exercée sur ledit réservoir S par ladite tige de piston 5 en fin de piquage. La force F2 s'ajoute au contraire à la force exercée par le ressort d'actionnement 6 sur la tige de piston 5 en début d'injection pour amplifier la force exercée sur ledit piston P par ladite tige de piston 5 en début d'injection. Le système de réglage de force peut exercer seulement la force F1, seulement la force F2, ou les deux forces F1 et F2.

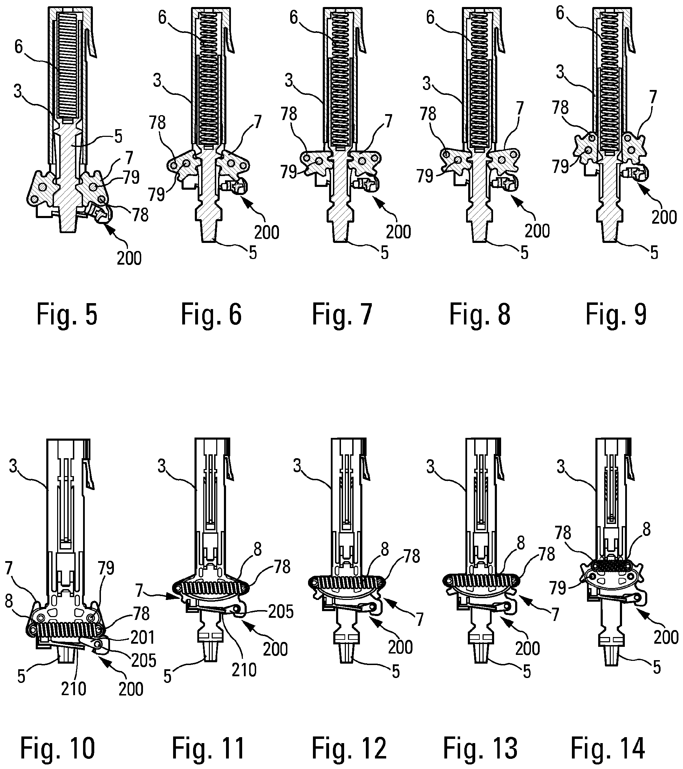

Les figures 5 à 14 illustrent les séquences d'actionnement de l'autoinjecteur de la figure 1.

Sur les figures 5 et 10, l'autoinjecteur est en position de repos avant actionnement. Dans cette position de repos, l'aiguille de la seringue S est disposée à l'intérieur du corps inférieur 1. Lesdits axes mobiles 78 sont disposés en avant desdits axes fixes 79 dans le sens de déplacement de la tige de piston 5, Le ressort d'actionnement 6 sollicite la tige de piston 5 en direction du piston de la seringue S, mais la tige de piston 5 est retenue dans la position de repos par la première projection 71 des organes pivotants 7 qui coopère avec le premier épaulement 51 de la tige de piston 5. Sous la pression du ressort d'actionnement 6, ledit premier épaulement 51 de la tige de piston 5 pousse la première projection 71 des organes pivotants 7 en rotation, mais selon l'invention, cette rotation est bloquée par un système de verrouillage 200.

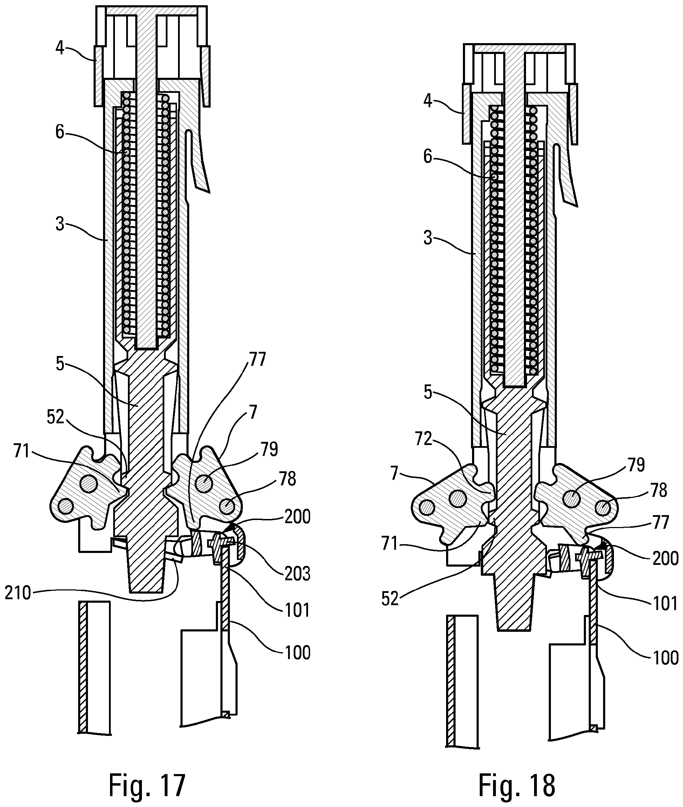

Les figures 2 à 4 et 15 à 18 illustrent plus particulièrement un système de verrouillage 200 selon un mode de réalisation avantageux de la présente invention. Ce système de verrouillage 200 comporte au moins un élément de verrouillage 201 déplaçable et/ou déformable entre une position de verrouillage, dans laquelle il bloque au moins un organe pivotant 7 en rotation, et une position de déverrouillage, dans laquelle ledit au moins un organe pivotant 7 peut pivoter. Dans l'exemple représenté, il n'y a qu'un seul élément de verrouillage 201 coopérant avec un seul organe pivotant 7, mais on pourrait prévoir un élément de verrouillage pour chaque organe pivotant. Ledit élément de verrouillage 201 est sollicité élastiquement vers sa position de verrouillage par un organe de sollicitation 210, tel qu'une lame élastique. Avantageusement, ledit élément de verrouillage 201 est monté pivotant sur ledit manchon 3 dudit corps, autour d'un axe de rotation 205. Dans l'exemple représenté, ledit élément de verrouillage 201 comporte une partie de blocage 202, qui coopère directement avec un organe pivotant 7, et une partie de commande 203 qui coopère avec un élément de déverrouillage 101 solidaire du manchon actionneur 100. De préférence, ledit élément de déverrouillage 101 est formé par une projection axiale dudit manchon actionneur 100. L'élément de déverrouillage pourrait être réalisé différemment.

Lorsque l'utilisateur veut utiliser l'autoinjecteur, il prend le dispositif, par exemple au niveau du corps supérieur 2 et il appuie le manchon actionneur 100 contre la partie de son corps où il veut réaliser l'injection. Le manchon actionneur 100 va alors se déplacer axialement vers l'intérieur du corps inférieur 1. Lorsque la course dudit manchon actionneur 100 vers l'intérieur du corps inférieur 1 est suffisante pour permettre la coopération entre l'élément de verrouillage 201 et l'élément de déverrouillage 101, l'élément de déverrouillage 101 va faire pivoter l'élément de verrouillage 201 du système de verrouillage autour de son axe 205, ce qui libère les organes pivotants 7. Si l'utilisateur relâche la pression sur le manchon actionneur 100 sans actionner l'autoinjecteur via le bouton d'actionnement 4, la lame élastique 210 va ramener l'élément de verrouillage 201 en position de verrouillage. Ceci permet notamment à l'utilisateur de choisir le site d'injection en testant plusieurs endroits sans actionner le bouton d'actionnement 4. Lorsque, après avoir déverrouillé les organes pivotants 7 par appui sur le manchon actionneur 100, l'utilisateur appuie sur le bouton axial d'actionnement 4, il déplace légèrement la tige de piston 5 axialement, ce qui provoque la rotation des organes pivotants 7 et l'actionnement de l'autoinjecteur.

Avantageusement, ledit élément de déverrouillage 101 coopère avec ledit système de verrouillage 200 en fin de course dudit manchon actionneur 100 entre sa position initiale de repos et sa position actionnée, notamment lorsque ledit manchon actionneur 100 a effectué au moins 90% de ladite course. Ceci permet de libérer l'actionnement de l'autoinjecteur seulement lorsqu'on est certain que le produit sera expulsé à la profondeur d'injection souhaitée, et d'éviter ainsi le risque d'un actionnement prématuré à une trop faible profondeur.

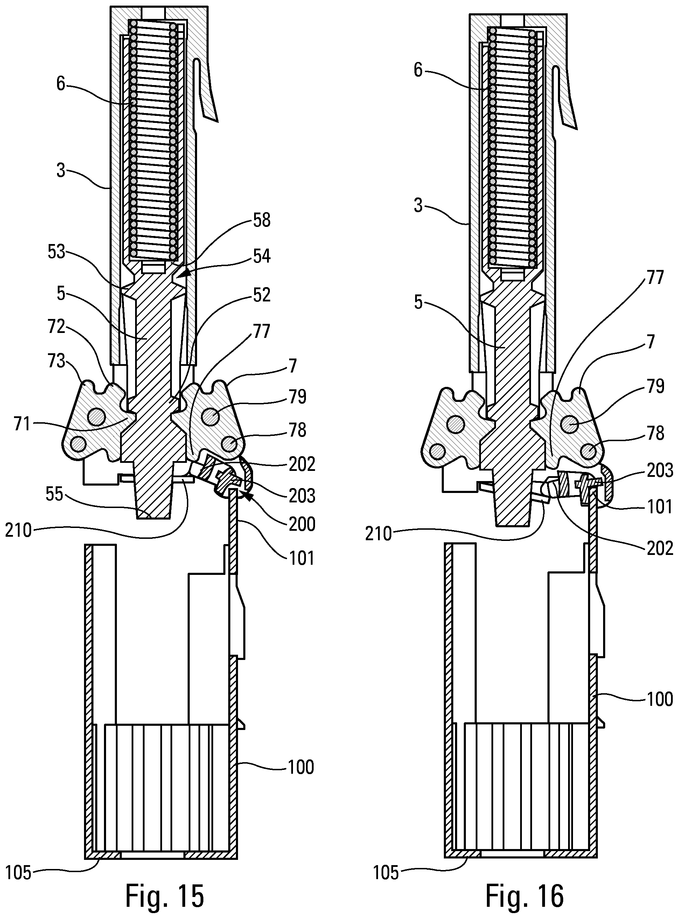

De par la géométrie de la partie de blocage 202 et de sa position par rapport à l'axe de rotation 205 de l'élément de verrouillage 201 du mode de réalisation représenté sur les figures 15 à 18, si l'utilisateur appuie sur le bouton axial 4 sans avoir déverrouillé le système de verrouillage 200, il renforce le blocage. Ce système de verrouillage est donc engageant, ce qui renforce la sécurité de l'actionnement.

La rotation des organes pivotants 7 générée par la force d'actionnement provoque le désengrènement entre la première projection 71 et le premier épaulement 51. Ceci libère donc la tige de piston 5, qui est alors déplacée axialement sous l'effet du ressort d'actionnement 6. Ceci provoque le déplacement de la seringue S dans le corps inférieur 1 et donc le piquage.

Lorsque la seconde projection 72 des organes pivotants atteint la surface avant de la seconde projection radiale 53 de la tige de piston 5, la phase de piquage n'est pas totalement terminée. Ceci est visible sur les figures 6 et 11. A ce moment-là, la surface avant de la seconde projection radiale 53 va faire tourner davantage les organes pivotant 7 en poussant sur leurs secondes projections 72. Ceci va encore davantage tendre ou charger les éléments élastiques 8, qui vont donc opposer une force croissante à la rotation des organes pivotants 7. Ceci génère une force de « freinage » ou « d'amortissement ». Ceci génère un amortissement en fin de piquage, en diminuant la force exercée par la tige de piston 5 sur la seringue S, ce qui améliore grandement le confort de l'utilisateur et évite d'endommager la collerette de la seringue S. Bien entendu, les forces du ressort d'actionnement 6 et des éléments élastiques 8 sont choisies de telle sorte que le piquage n'est qu'amortit sans être stoppé.

Au fur et à mesure que les organes pivotants 7 tournent lors de la phase de piquage, les éléments élastiques 8 se tendent de plus en plus. Simultanément, les axes mobiles 78 des organes pivotants 7 se rapprochent progressivement des axes fixes 79. Le dispositif est ajusté avantageusement pour générer un couple maximal au moment de (ou juste avant) la fin de la phase de piquage. Le point neutre dans lequel les axes mobiles et fixes sont alignés peut donc être atteint au moment de (ou juste avant) la fin de la phase de piquage.

Lorsque les organes pivotants 7 et les éléments élastiques 8 sont dans la position neutre, représentée sur les figures 7 et 12, la tige de piston est toujours sollicitée axialement par le ressort d'actionnement 6. Cette position neutre n'est donc pas stable, et le système bascule automatiquement de l'état de freinage du piquage vers l'état d'amplification du début de l'injection. Eventuellement, la troisième projection 73 peut être disposée par rapport à la seconde projection 72 de telle sorte que les organes pivotants 7 effectuent juste après le passage de la position neutre une légère rotation sous l'effet des éléments élastiques 8 tendus. Ceci peut permettre de générer un son audible lorsque ladite troisième projection 73 va heurter la seconde projection radiale 53 de la tige de piston, pour indiquer à l'utilisateur le début de la phase d'injection.

Lorsque l'aiguille atteint sa position de piquage avec une insertion complète de l'aiguille, la phase d'injection est déclenchée, ce qui est représenté sur les figures 8 et 13. L'extrémité avant 55 de la tige de piston va alors pousser sur le piston sous l'effet de la force exercée par le ressort d'actionnement 6. Pendant toute la phase d'injection, la tige de piston 5 coulisse à l'intérieur de la seringue S en poussant le piston de celle-ci sous l'effet du ressort 6. Le produit est ainsi distribué à travers l'aiguille.

Au début de la phase d'injection, la troisième projection 73 de chaque organe pivotant 7 va donc venir en contact de la surface arrière de la seconde projection radiale 53. Comme en fin de phase de piquage, le couple exercé par le système est maximal juste après la position neutre, et les éléments élastiques 8 tendus sollicitent donc fortement les organes pivotants 7 en rotation. Ceci a pour effet d'amplifier la force du ressort d'actionnement 6 en début de phase d'injection. Cette amplification augmente la force exercée par la tige de piston 5 sur le piston, et permet ainsi de garantir le décollement du piston de sa position de repos, sans avoir à augmenter la force du ressort d'actionnement 6. En effet, la résistance maximale lors de la phase d'injection est créée au moment du décollement du piston. Une fois que l'injection a débutée, les frottements du piston dans la seringue S, la viscosité du produit à injecter et la résistance du passage étroit de l'aiguille sont inférieurs et ne nécessitent donc plus la même force du ressort d'actionnement 6.

Comme visible sur les figures 9 et 14, les organes pivotants 7 se désengrènent de la tige de piston après une course d'injection relativement petite de la tige de piston 5, typiquement de quelques millimètres, par exemple environ 4 mm. A partir de ce désengrènement, le système devient inactif, et l'injection du produit se poursuit normalement. Il est envisageable d'adapter le système de réglage de force pour qu'il puisse amplifier l'effort exercé sur le piston pendant une partie de course d'injection plus grande, par exemple environ 20 mm, voire pendant la totalité de la course d'injection, notamment avec des réservoirs dont la dimension axiale serait réduite.

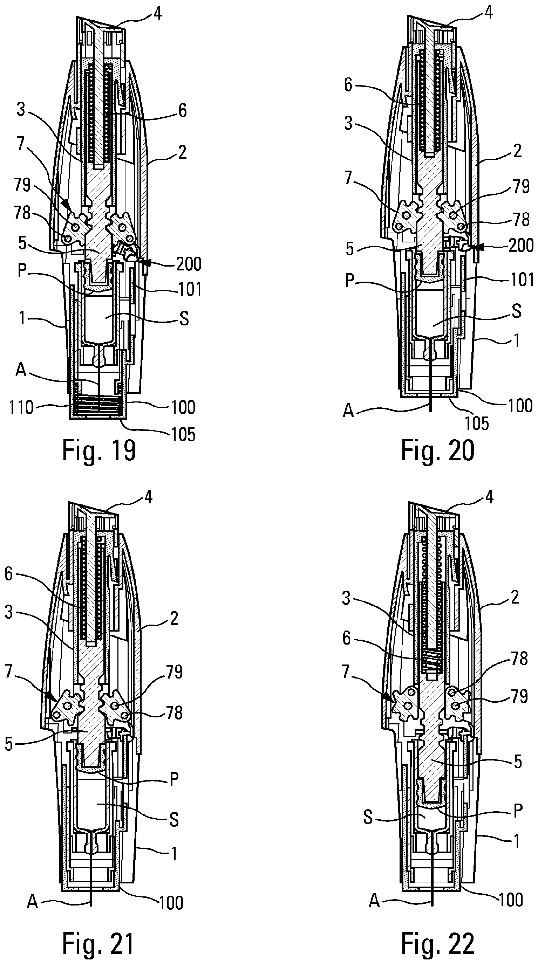

Les figures 19 à 22 illustrent un second mode de réalisation avantageux de l'autoinjecteur dans lequel il n'y a pas de piquage automatique, ou auto-piquage. Il est à noter que ces figures ne sont que schématiques, et non limitatives d'un tel mode de réalisation. Le ressort d'actionnement 6 ne réalise dans ce mode de réalisation que l'injection en déplaçant la tige de piston 5, et donc le piston P, entre la position de repos et la position d'injection. Ici, le piquage est réalisé manuellement, au moyen du manchon actionneur 100. Dans ce mode de réalisation, la seringue S est donc fixe par rapport au corps de l'autoinjecteur.

Dans la position de repos illustrée sur la figure 19, le manchon actionneur 100, sollicité par son ressort 110, entoure l'aiguille A de la seringue S. lorsque l'utilisateur veut actionner l'autoinjecteur, il place la surface d'extrémité axiale 105 du manchon actionneur 100 contre le site d'injection, et il appuie sur l'autoinjecteur. Le manchon actionneur 100 va alors coulisser axialement vers l'intérieur du corps inférieur 1, exposant ainsi l'aiguille A de la seringue S qui va alors pénétrer dans le corps de l'utilisateur, comme visible sur la figure 20. L'utilisateur peut alors actionner le bouton axial 4 pour déplacer la tige de piston 5, et le système de réglage de force devient alors actif comme décrit précédemment, en exerçant, via les organes pivotants 7, une force d'amplification F2 sur la tige de piston 5 en début d'injection, comme illustré sur la figure 21. La figure 22 montre les organes pivotants 7 désengrenés de la tige de piston 5, la fin de l'injection se poursuivant alors sans action desdits organes pivotants. En variante, on pourrait envisager de faire agir les organes pivotants pendant toute la phase d'injection.

Le système de verrouillage 200 est identique au premier mode de réalisation décrit précédemment, et il ne sera donc pas décrit à nouveau ici.

Dans ce mode de réalisation, le système de réglage de force n'exerce donc que la force d'amplification F2 en début d'injection, et n'intervient pas pendant le piquage.

Typiquement, des forces F1 d'amortissement et/ou F2 d'amplification de l'ordre de 30N sont possibles. Bien entendu, d'autres valeurs d'amortissement et de freinage peuvent être obtenues en choisissant de manière appropriée les éléments élastiques 8 et en dimensionnant de manière appropriée les organes pivotants 7.

La présente invention s'applique à des dispositifs utilisés notamment pour les traitements des maladies auto-immunes, par exemple du type arthrites rhumatoïdes, scléroses multiples, maladie de Crohn, pour les traitements contre le cancer, pour les traitements antiviraux, par exemple du type hépatites, pour les traitements contre le diabète, pour les traitements contre l'anémie ou pour les traitements de crises, par exemple en cas de choc anaphylactique.

Bien que la présente invention ait été décrite en référence à deux modes de réalisation avantageux, il est entendu que diverses modifications sont possibles pour l'homme du métier sans sortir du cadre de la présente invention tel que défini par les revendications annexées.

## Revendications

1. Autoinjecteur comportant un corps (1, 2), un réservoir (S) contenant du produit fluide et comportant un piston et une aiguille, tel qu'une seringue pré-remplie, ledit autoinjecteur comportant une tige de piston (5) adaptée à coopérer avec le piston dudit réservoir (S), ladite tige de piston (5) étant déplaçable entre une position de repos et une position d'injection dans laquelle ladite tige de piston (5) a déplacé le piston du réservoir (S) pour injecter le produit fluide à travers l'aiguille, un ressort d'actionnement (6) étant prévu pour solliciter ladite tige de piston (5) vers sa position d'injection, l'autoinjecteur comprenant un système de réglage de force (7, 8) adapté à exercer au moins une force (F2) sur ladite tige de piston (5), ladite force (F2) s'ajoutant à la force exercée par ledit ressort d'actionnement (6) sur ladite tige de piston (5) en début d'injection pour amplifier la force exercée sur ledit piston par ladite tige de piston (5) en début d'injection, ledit système de réglage de force (7, 8) comprenant deux organes pivotants (7) coopérant avec ladite tige de piston (5), lesdits organes pivotants (7) étant reliés entre eux par deux éléments élastiques (8), au moins un organe pivotant (7) étant bloqué avant actionnement par un système de verrouillage (200), bloquant ainsi l'actionnement de l'autoinjecteur, ledit autoinjecteur comportant un élément de déverrouillage (101) adapté à déplacer et/ou déformer ledit système de verrouillage (200) pour débloquer ledit au moins un organe pivotant (7), et permettre ainsi l'actionnement de l'autoinjecteur.

2. Autoinjecteur selon la revendication 1, dans lequel ledit corps (1, 2) comporte un manchon (3), lesdits organes pivotants (7) étant monté pivotants sur ledit manchon (3) autour d'axes de rotation (79) parallèles.

3. Autoinjecteur selon la revendication 2, dans lequel lesdits éléments élastiques (8) sont fixés auxdits organes pivotants (7) au niveau d'axes mobiles parallèles (78), tels que des tiges ayant deux bords latéraux, formés sur lesdits organes pivotants (7).

4. Autoinjecteur selon la revendication 3, dans lequel, lorsque la tige de piston (5) se déplace vers sa position d'injection, lesdits axes mobiles (78) sont disposés en arrière desdits axes fixes (79) dans le sens de déplacement de ladite tige de piston (5), lesdits éléments élastiques chargés (8), en début de déplacement de la tige de piston (5) vers sa position d'injection, faisant tourner lesdits organes pivotants (7) de telle manière à détendre lesdits éléments élastiques (8), créant ainsi une force d'amplification (F2) en début d'injection.

5. Autoinjecteur selon l'une quelconque des revendications précédentes, dans lequel chaque organe pivotant (7) comprend plusieurs projections (71, 72, 73) adaptées à coopérer avec plusieurs projections radiales (52, 53) de la tige de piston (5).

6. Autoinjecteur selon l'une quelconque des revendications précédentes, dans lequel ledit autoinjecteur est actionné par un bouton axial (4).

7. Autoinjecteur selon l'une quelconque des revendications précédentes, dans lequel, avant injection, ladite tige de piston (5) est d'abord déplacée par ledit ressort d'actionnement (6) entre ladite position de repos et une position de piquage, dans laquelle ladite tige de piston (5) a déplacé ledit réservoir (S) par rapport audit corps (1, 2) pour réaliser le piquage.

8. Autoinjecteur selon la revendication 7, dans lequel lors du piquage, la tige de piston (5) coopère avec le piston du réservoir (S) pour déplacer ledit réservoir (S) par rapport au corps (1, 2).

9. Autoinjecteur selon les revendications 2, 3 et 7, dans lequel, lorsque la tige de piston (5) se déplace de sa position de repos vers sa position de piquage, lesdits axes mobiles (78) sont disposés en avant desdits axes fixes (79) dans le sens de déplacement de ladite tige de piston (5), ladite tige de piston (5), en fin de déplacement vers sa position de piquage, faisant tourner lesdits organes pivotants (7) de telle manière à charger lesdits éléments élastiques (8), créant ainsi une force de freinage (F1) en fin de piquage, ladite force (F1) étant opposée à la force exercée par ledit ressort d'actionnement (6) sur ladite tige de piston (5) en fin de piquage pour diminuer la force exercée sur ledit réservoir (S) par ladite tige de piston (5) en fin de piquage.

10. Autoinjecteur selon l'une quelconque des revendications précédentes, dans lequel ledit système de verrouillage (200) comporte au moins un élément de verrouillage (201) déplaçable et/ou déformable entre une position de verrouillage, dans laquelle il bloque au moins un organe pivotant (7) en rotation, et une position de déverrouillage, dans laquelle ledit au moins un organe pivotant (7) peut pivoter.

11. Autoinjecteur selon la revendication 10, dans lequel ledit au moins un élément de verrouillage (201) est sollicité élastiquement vers sa position de verrouillage par un organe de sollicitation (210), tel qu'une lame élastique.

12. Autoinjecteur selon la revendication 10 ou 11, dans lequel ledit au moins un élément de verrouillage (201) est monté pivotant sur un manchon (3) dudit corps (1, 2), autour d'un axe de rotation (205).

13. Autoinjecteur selon l'une quelconque des revendications 10 à 12, dans lequel ledit au moins un élément de verrouillage (201) comporte une partie de blocage (202) qui coopère directement avec un organe pivotant (7) et une partie de commande (203) qui coopère avec ledit élément de déverrouillage (101).

14. Autoinjecteur selon l'une quelconque des revendications précédentes, dans lequel ledit élément de déverrouillage (101) est formé sur un manchon actionneur (100), monté coulissant dans ledit corps qui comporte un corps inférieur (1) et un corps supérieur (2), et dont une surface d'extrémité axiale (105) est en contact avec la zone où l'injection doit avoir lieu, ledit manchon actionneur (100) étant adapté à coulisser par rapport audit corps inférieur (1) entre une position initiale de repos, avant actionnement, dans laquelle il se projette axialement hors dudit corps inférieur (1), une position actionnée, dans laquelle il est déplacé axialement vers l'intérieur dudit corps inférieur (1), et une position finale de sécurité, dans laquelle il est à nouveau projeté hors dudit corps inférieur (1), pour recouvrir l'aiguille de la seringue (S) après injection, ledit manchon actionneur (100) étant sollicité axialement vers l'extérieur dudit corps inférieur (1) par un ressort (110).

15. Autoinjecteur selon la revendication 14, dans lequel ledit élément de déverrouillage (101) coopère avec ledit système de verrouillage (200) en fin de course dudit manchon actionneur (100) entre sa position initiale de repos et sa position actionnée, notamment lorsque ledit manchon actionneur (100) a effectué au moins 90% de ladite course.

## Patentansprüche

1. Autoinjektor, umfassend einen Körper (1, 2), einen Behälter (S), der ein fluides Produkt enthält und einen Kolben und eine Nadel, wie eine vorgefüllte Spritze, umfasst, wobei der Autoinjektor eine Kolbenstange (5) umfasst, die dafür geeignet ist, mit dem Kolben des Behälters (S) zusammenzuwirken, wobei die Kolbenstange (5) zwischen einer Ruheposition und einer Injektionsposition verschiebbar ist, in der die Kolbenstange (5) den Kolben des Behälters (S) verschoben hat, um das fluide Produkt über die Nadel zu injizieren, wobei eine Betätigungsfeder (6) vorgesehen ist, um die Kolbenstange (5) in ihre Injektionsposition vorzuspannen, wobei der Autoinjektor ein Kraftregelungssystem (7, 8) umfasst, welches dazu geeignet ist, mindestens eine Kraft (F2) auf die Kolbenstange (5) auszuüben, wobei die Kraft (F2) zu der Kraft hinzukommt, die zu Beginn der Injektion von der Betätigungsfeder (6) auf die Kolbenstange (5) ausgeübt wird, um die von der Kolbenstange (5) zu Beginn der Injektion auf den Kolben ausgeübte Kraft zu verstärken, wobei das Kraftregelungssystem (7, 8) zwei schwenkbare Organe (7) umfasst, die mit der Kolbenstange (5) zusammenwirken, wobei die schwenkbaren Organe (7) durch zwei elastische Elemente (8) miteinander verbunden sind, wobei mindestens ein schwenkbares Organ (7) vor der Betätigung durch ein Verriegelungssystem (200) blockiert wird, wodurch die Betätigung des Autoinjektors blockiert wird, wobei der Autoinjektor ein Entriegelungselement (101) umfasst, welches dazu geeignet ist, das Verriegelungssystem (200) zu verschieben und/oder zu verformen, um das mindestens eine schwenkbare Organ (7) zu entsperren und so die Betätigung des Autoinjektors zu ermöglichen.

2. Autoinjektor nach Anspruch 1, wobei der Körper (1, 2) eine Muffe (3) umfasst, wobei die schwenkbaren Organe (7) auf der Muffe (3) um parallele Drehachsen (79) schwenkbar gelagert sind.

3. Autoinjektor nach Anspruch 2, wobei die elastischen Elemente (8) an mobilen parallelen Achsen (78), wie Stangen mit zwei seitlichen Rändern, die auf den schwenkbaren Organen (7) ausgebildet sind, an den schwenkbaren Organen (7) befestigt sind.

4. Autoinjektor nach Anspruch 3, wobei, wenn sich die Kolbenstange (5) in ihre Injektionsposition verschiebt, die mobilen Achsen (78) in Verschiebungsrichtung der Kolbenstange (5) hinter den feststehenden Achsen (79) angeordnet werden, wobei die elastischen belasteten Elemente (8) zu Beginn der Verschiebung der Kolbenstange (5) in ihre Injektionsposition die schwenkbaren Organe (7) derart drehen, dass die elastischen Elemente (8) gelockert werden, wodurch eine Verstärkungskraft (F2) zu Beginn der Injektion erzeugt wird.

5. Autoinjektor nach einem der vorhergehenden Ansprüche, wobei jedes schwenkbare Organ (7) mehrere Vorsprünge (71, 72, 73) umfasst, die dazu geeignet sind, mit mehreren radialen Vorsprüngen (52, 53) der Kolbenstange (5) zusammenzuwirken.

6. Autoinjektor nach einem der vorhergehenden Ansprüche, wobei der Autoinjektor durch einen axialen Knopf (4) betätigt wird.

7. Autoinjektor nach einem der vorhergehenden Ansprüche, wobei die Kolbenstange (5) vor der Injektion zunächst durch die Betätigungsfeder (6) zwischen der Ruheposition und einer Stechposition verschoben wird, in der die Kolbenstange (5) den Behälter (S) in Bezug auf den Körper (1, 2) verschoben hat, um das Stechen auszuführen.

8. Autoinjektor nach Anspruch 7, wobei die Kolbenstange (7) während des Stechens mit dem Kolben des Behälters (S) zusammenwirkt, um den Behälter (S) in Bezug auf den Körper (1, 2) zu verschieben.

9. Autoinjektor nach den Ansprüchen 2, 3 und 7, wobei, wenn sich die Kolbenstange (5) von ihrer Ruheposition in ihre Stechposition verschiebt, die mobilen Achsen (78) in Verschiebungsrichtung der Kolbenstange (5) vor den feststehenden Achsen (79) angeordnet sind, wobei die Kolbenstange (5) am Ende der Verschiebung in ihre Stechposition die schwenkbaren Organe (7) derart dreht, dass die elastischen Elemente (8) belastet werden, so dass eine Bremskraft (F1) am Ende des Stechens erzeugt wird, wobei die Kraft (F1) der von der Betätigungsfeder (6) auf die Kolbenstange (5) am Ende des Stechens ausgeübten Kraft entgegengesetzt ist, um die von der Kolbenstange (5) am Ende des Stechens auf den Behälter (S) ausgeübte Kraft zu verringern.

10. Autoinjektor nach einem der vorhergehenden Ansprüche, wobei das Verriegelungssystem (200) mindestens ein Verriegelungselement (201) umfasst, welches zwischen einer Verriegelungsposition, in der es mindestens ein schwenkbares Organ (7) in seiner Drehung blockiert, und einer Entriegelungsposition, in der das mindestens eine schwenkbare Organ (7) sich verschwenken kann, verschiebbar und/oder verformbar ist.

11. Autoinjektor nach Anspruch 10, wobei das mindestens eine Verriegelungselement (201) durch ein Vorspannorgan (210), wie eine elastische Lamelle, elastisch in seine Verriegelungsposition vorgespannt ist.

12. Autoinjektor nach Anspruch 10 oder 11, wobei das mindestens eine Verriegelungselement (201) um eine Drehachse (205) schwenkbar auf einer Muffe (3) des Körpers (1, 2) gelagert ist.

13. Autoinjektor nach einem der Ansprüche 10 bis 12, wobei das mindestens eine Verriegelungselement (201) ein Blockierteil (202), das direkt mit einem schwenkbaren Organ (7) zusammenwirkt, und ein Steuerteil (203) umfasst, das mit dem Entriegelungselement (101) zusammenwirkt.

14. Autoinjektor nach einem der vorhergehenden Ansprüche, wobei das Entriegelungselement (101) auf einer Betätigungsmuffe (100) ausgebildet ist, die gleitend in dem Körper gelagert ist, der einen unteren Körper (1) und einen oberen Körper (2) umfasst und deren eine axiale Endfläche (105) in Kontakt mit dem Bereich ist, in dem die Injektion stattfinden soll, wobei die Betätigungsmuffe (100) dafür geeignet ist, in Bezug auf den unteren Körper (1) zwischen einer anfänglichen Ruheposition vor der Betätigung, in der sie axial aus dem unteren Körper (1) vorsteht, einer betätigten Position, in der sie axial zum Inneren des unteren Körpers (1) verschoben ist, und einer gesicherten Endposition, in der sie wieder aus dem unteren Körper (1) vorsteht, um die Nadel der Spritze (S) nach der Injektion wieder abzudecken, zu gleiten, wobei die Betätigungsmuffe (100) durch eine Feder (110) axial zum Äußeren des unteren Körpers (1) vorgespannt ist.

15. Autoinjektor nach Anspruch 14, wobei das Entriegelungselement (101) am Ende des Weges der Betätigungsmuffe (100) zwischen ihrer anfänglichen Ruheposition und ihrer betätigten Position mit dem Verriegelungssystem (20) zusammenwirkt, insbesondere, wenn die Betätigungsmuffe (100) mindestens 90 % des Weges zurückgelegt hat.

## Claims

1. An autoinjector comprising a body (1, 2), and a reservoir (S) containing fluid and including a piston and a needle, such as a prefilled syringe, said autoinjector further comprising a piston rod (5) that is adapted to co-operate with the piston of said reservoir (S), said piston rod (5) being movable between a rest position and an injection position in which said piston rod (5) has moved the piston of the reservoir (S) so as to inject the fluid through the needle, an actuator spring (6) being provided so as to urge said piston rod (5) towards its injection position, the autoinjector further comprising a force-adjustment system (7, 8) that is adapted to exert at least one force (F2) on said piston rod (5), said force (F2) adding to the force exerted by said actuator spring (6) on said piston rod (5) at the beginning of injection, so as to amplify the force exerted on said piston by said piston rod (5) at the beginning of injection, said force-adjustment system (7, 8) comprising two pivot members (7) that co-operate with said piston rod (5), said pivot members (7) being connected together by two resilient elements (8), at least one pivot member (7) being blocked prior to actuation by a locking system (200), thus preventing the autoinjector from being actuated, said autoinjector further comprising an unlocking element (101) that is adapted to move and/or to deform said locking system (200) so as to unblock said at least one pivot member (7), and thus make it possible to actuate the autoinjector.

2. An autoinjector according to claim 1, wherein said body (1, 2) includes a sleeve (3), said pivot members (7) being mounted to pivot on said sleeve (3) about pivot pins (79) that are parallel.

3. An autoinjector according to claim 2, wherein said resilient elements (8) are fastened to said pivot members (7) via parallel movable pins (78), such as rods having two side edges, that are formed on said pivot members (7).

4. An autoinjector according to claim 3, wherein, when the piston rod (5) moves towards its injection position, said movable pins (78) are arranged behind said stationary pins (79) in the travel direction of said piston rod (5), said loaded resilient elements (8), at the beginning of travel of the piston rod (5) towards its injection position, causing said pivot members (7) to pivot in such a manner as to relax said resilient elements (8), thereby creating an amplification force (F2) at the beginning of injection.

5. An autoinjector according to any preceding claim, wherein each pivot member (7) includes a plurality of projections (71, 72, 73) that are adapted to co-operate with a plurality of radial projections (52, 53) of the piston rod (5).

6. An autoinjector according to any preceding claim, wherein said autoinjector is actuated by an axial button (4).

7. An autoinjector according to any preceding claim, wherein, prior to injection, said piston rod (5) is initially moved by said actuator spring (6) between said rest position and a pricking position in which said piston rod (5) has moved said reservoir (S) relative to said body (1, 2) so as to perform pricking.

8. An autoinjector according to claim 7, wherein, during pricking, the piston rod (5) co-operates with the piston of the reservoir (S) so as to move said reservoir (S) relative to the body (1, 2).

9. An autoinjector according to claims 2, 3 and 7, wherein, when the piston rod (5) moves from its rest position towards its pricking position, said movable pins (78) are arranged in front of said stationary pins (79) in the travel direction of said piston rod (5), said piston rod (5), at the end of travel towards its pricking position, causing said pivot members (7) to pivot so as to load said resilient elements (8), thereby creating a braking force (F1) at the end of pricking, said force (F1) being opposed, at the end of pricking, to the force exerted by said actuator spring (6) on said piston rod (5), so as to decrease the force exerted on said reservoir (S) by said piston rod (5) at the end of pricking.

10. An autoinjector according to any preceding claim, wherein said locking system (200) includes at least one locking element (201) that is movable and/or deformable between a locked position in which it prevents at least one pivot member (7) from pivoting, and an unlocked position in which said at least one pivot member (7) can pivot.

11. An autoinjector according to claim 10, wherein said at least one locking element (201) is urged resiliently towards its locked position by a drive member (210), such as a resilient blade.

12. An autoinjector according to claim 10 or claim 11, wherein said at least one locking element (201) is mounted to pivot on a sleeve (3) of said body (1,2), about a pivot pin (205).

13. An autoinjector according to any one of claims 10 to 12, wherein said at least one locking element (201) includes a blocking portion (202) that co-operates directly with a pivot member (7), and a control portion (203) that co-operates with said unlocking element (101).

14. An autoinjector according to any preceding claim, wherein said unlocking element (101) is formed on an actuator sleeve (100) that is slidably mounted in said body including a lower body (1) and an upper body (2), and that has an axial end surface (105) that is in contact with the zone in which injection is to take place, said actuator sleeve (100) being adapted to slide relative to said lower body (1) between an initial rest position in which it projects axially out from said lower body (1) prior to actuation, an actuated position in which it is moved axially towards the inside of said lower body (1), and a final safety position in which it once again projects out from said lower body (1), so as to cover the needle of the syringe (S) after injection, said actuator sleeve (100) being urged axially towards the outside of said lower body (1) by a spring (110).

15. An autoinjector according to claim 14, wherein said unlocking element (101) co-operates with said locking system (200) at the end of stroke of said actuator sleeve (100), between its initial rest position and its actuated position, in particular when said actuator sleeve (100) has performed at least 90% of said stroke.
